# EUROPEAN PATENT APPLICATION

(11) **EP 4 053 554 A1**
(43) Date of publication of application: **07.09.2022**
(21) Application number: 21160260.2
(22) Date of filing: 02.03.2021
(51) Int. Cl.: G01N 33/487

(54) **ARRANGEMENT FOR ANALYZING A LIQUID SAMPLE**

(71) Applicant: EXIAS Medical GmbH, 8020 Graz (AT)
(72) Inventor: Schlaminger, Michael, 8010 Graz (AT); Steinböck, Wolf-Dietrich, 8044 Graz (AT); Hofer, Anton, 8505 St. Nikolai i. Sausal (AT)
(74) Representative: Dilg, Haeusler, Schindelmann Patentanwaltsgesellschaft mbH

(57) **Abstract**

It is provided an arrangement (1) and a method for measuring at least one constituent of a liquid sample (47), in particular comprising serum, plasma and/or urine, the arrangement comprising: a first portion (24) adapted to partly delimit a reception space (7) for the sample at a first side (40) and comprising at least one measurement area (21); a second portion (22) comprising: a reception space wall section (49), the second portion being coupled to the first portion (24) such that the reception space wall section (49) partly delimits the reception space (7) at a second side (50); and a sample input cup (4) delimiting a sample input region (8) into which the sample is fillable, wherein the sample input region (8) is in fluid communication with the reception space (7),
wherein the reception space wall section (49) and at least a portion of the sample input cup (4) are integrally formed.

## Description

### Field of invention

The present invention relates to an arrangement and to a method of measuring at least one constituent of a liquid sample, in particular comprising serum, plasma and/or urine. Furthermore, the present invention relates to a method for manufacturing such an arrangement.

### Art Background

The document EP 3 139 162 A1 discloses a movable measurement cell for measuring at least one constituent of a liquid sample comprising a first portion and a second portion as well as a sample supply system attached to an inlet to allow supply of a liquid sample into a reception space partly provided by the second portion and/or first portion.

Conventionally, measurement systems for measuring of electrolytes in liquids are known. Thereby, an amount of a sample to be analyzed may be applied to individual devices with different specific electrodes or a particular amount of a sample may be applied to multiple electrodes combined in one apparatus to determine multiple ion activities. However, conventional measurement devices typically use large and complex configurations to provide the appropriate fluid flow across multiple electrodes. Further, in conventional systems a relatively large amount of a sample solution and/or a complex fluidic and pump system may be required to perform accurate measurements and/or perform required preparation steps.

Thus, there may be a need for an arrangement for measuring a constituent of a liquid sample, wherein at least some of the aforementioned problems are mitigated. In particular, there may be a need for a measuring arrangement which is less complex than conventionally known and/or does not require extensive amounts of sample, while ensuring accurate measurement of constituents of the sample. Moreover, a respective manufacturing method for manufacturing the arrangement may be required. Furthermore, a respective method for measuring a constituent of a liquid sample may be needed, which in particular generates accurate measurement results for a small amount of liquid sample.

### Summary of the Invention

This need may be met by the subject-matter according to the independent claims which are directed to an arrangement for measuring at least one constituent of a liquid sample, a method for measuring at least one constituent of a liquid sample and a method for manufacturing the arrangement, respectively.

According to an embodiment of the present invention it is provided an aarrangement according to claim 1 for measuring at least one constituent of a liquid sample, in particular comprising serum, plasma and/or urine, the arrangement comprising: a first portion adapted to partly delimit a reception space for the sample at a first side and comprising at least one measurement area; a second portion comprising: a reception space wall section, the second portion being coupled to the first portion such that the reception space wall section partly delimits the reception space at a second side; and a sample input cup delimiting a sample input region into which the sample is fillable, wherein the sample input region is in fluid communication with the reception space, wherein the reception space wall section and at least a portion of the sample input cup are integrally formed.

The arrangement for measuring at least one constituent of a liquid sample may comprise a measurement cell formed by the first portion and the second portion coupled together. The aarrangement (also referred to as measurement system or apparatus) may in particular be adapted to measure the concentration of at least one ion, such as K⁺, Ca⁺⁺, Na⁺, Cl⁻, and/or pH and/or HCT and/or partial pressures of O₂, CO₂ and/or concentrations of Glucose, Lactate, Urea, Creatinine or the like. For each particular analyte, at least one separate respective sensitive (measurement) area may be provided. When several sensitive areas for measurement of different constituents of the liquid are provided, the sensitive area may be arranged side by side along a longitudinal direction of the reception space. The reception space may also be referred to as measurement channel.

The measurement system may be adapted to perform potentiometric and/or amperometric measurement and/or conductometric measurements i.e. measurements of electrical potentials and/or electric currents and/or electric impedances. As measurement result, concentration of different analytes may be determined and/or the values of the partial pressures of different gases within the sample may be determined and/or the volumetric amount of blood cells. The measurement system may alternatively or additionally also be adapted to perform optical measurements.

The reception space may in particular be configured as a channel having a longitudinal direction corresponding to a flow direction during supply of the sample into the reception space. The reception space (at least partly) enclosed by parts of the first portion and the second portion may be formed as a channel or as a groove. The reception space may be accessed after connecting the second portion to the first portion via the sample input cup and one or more further conduit sections.

The second portion may be integrally formed and may not comprise any electrically conducting material. The first portion may be glued to the second portion or may be connected by other means. A seal may be arranged between interfaces of the first portion and the second portion in order to liquid tightly seal the reception space.

The measurement cell, in particular formed by the first portion and the second portion which are coupled to each other, may be configured as a consumable, which may be replaced from time to time (for example after having performed a predetermined number of measurements or after a predefined time interval or at regular frequency). The measurement cell may be inserted for example into a reception space of a measuring apparatus. The measuring apparatus may have processing capabilities and control capabilities for processing measurement results from the at least one measurement area and/or also to control further components of the arrangement or the measurement apparatus, such as a liquid transportation system comprising one or more pumps.

The first portion and the second portion may separately be manufactured. The first portion may be configured as a substantially plane plate comprising one or more analyte sensitive areas, such as one or more optodes, one or more metabolite sensors, one or more electrodes, and/or one or more amperometric sensors, depending on the application. The first portion may comprise conductors electrically connected to the one or more measurement areas. When coupled or inserted into a reception space of a measurement apparatus, these conductors may electrically be connected to processing or control equipment such as to allow acquiring measurement data from the at least one measurement area. The first portion may comprise further electronic circuitry as required.

The second portion may not comprise any electrical conductor. When the first portion and the second portion are coupled to each other, the reception space for the sample may be surrounded at the first side by portions of the first portion and at the second side by the reception space wall section of the second portion.

The measurement may take place when the sample is filled into the reception space where the measurement area(s) may come in contact with the sample. The sample may be filled into the reception space, after the sample has been filled into the sample input cup, namely into the sample input region. During normal operation for preparing a measurement or performing a measurement of the liquid sample, the arrangement may have a particular orientation, such that a longitudinal axis of the sample input cup is oriented substantially vertically. The sample input cup may laterally delimit the sample input region, but may allow access to the sample input region from above, in particular via the sample input aperture.

After the sample has been applied into the sample input region within the sample input cup, the sample may for example be conveyed into the reception space for measurement by applying or exerting an negative pressure (suction) in a region downstream the reception space, in particular at a reception space output port as will be described below. Via the sample input cup, a sample to be measured and/or a wash solution and/or a calibration solution may be filled during different steps of performing a measurement and/or performing maintenance of the arrangement for measuring the constituent or the measuring apparatus or for preparation of a measurement.

When the reception space wall section of the second portion at least a portion of the sample input cup are integrally formed, problems regarding contamination of surfaces exposed to sample and/or calibration solution and/or washing solution may be mitigated. In particular, an inner face of the sample input cup may have a smooth surface, and may not have any gaps or spaces between connected components into which potentially sample and/or calibration solution and/or washing solution may penetrate, leading to contamination, as is observed in conventional systems.

Embodiments of the present invention provide a highly integrated apparatus and method for determination of at least one constituent of a liquid sample, typically serum, plasma and urine, in particular in the environment of a clinical chemistry analyzer. Further, embodiments of the present invention provide a method for manufacturing a highly integrated measurement cell with rotationally symmetrical widening of the input side of the measuring channel which serves as a sample compartment.

The arrangement may automatically analyse a chemical composition contained in, respectively at least one constituent of, for example, serum, plasma, or urine or other biologically important substances (called analytes) in body fluids. As one of the biochemical test arrays, the chemical analyzer measures the concentration and/or activity of a substance to be measured. The arrangement may be configured as CC-Analyzer (clinical chemistry analyzer) may be used in a hospital laboratory.

The measurement of the electrolytes, in particular sodium, potassium, chloride, in body fluids is one of the most frequently performed measurements as the electrolytes are of major physiological importance for numerous functions of the body.

Embodiments of the present invention may support analysis of clinical samples such as serum and urine in ion selective electrode (ISE) devices, involving performing multiple tests of a test solution using a variety of ion-specific analyzer electrodes. In general, an analyzer electrode (an example of a measurement area) reacts to a certain ion activity on constituents of a sample. Typically, the sample and operating solutions may be positioned in front of ion-sensitive electrodes, the electromotive force (emf) is measured and processed, and the results may reported to the user.

Embodiments of the present invention may support one of two different technologies, namely direct ISE and indirect ISE.

In direct ISE the patient's serum sample is brought into direct contact with the electrode surface and the activity of the relevant ion is measured in the plasma water of the serum. Indirect ISE, on the other hand, involves diluting the patient's serum with a buffer before the sample encounters the ISE membranes. The use of diluted test solutions reduces the amount of test solution required, i.e. sample volume and reduces the harmful effects of constituent such as protein, which may be present in high concentrations in some test solutions. This method may then calculate the electrolyte concentration in the diluted sample by assuming that the original sample had a plasma water concentration of 93%. Indirect ISE analyzers use this calculation because electrolytes are distributed only in the aqueous phase of plasma, while dissolved solids-mostly proteins and lipids-typically make up the remaining 7% of a patient's plasma volume. Indirect ISE may compensate for the presence of solids (proteins/lipids) in a sample. If a sample contains more or less than 7% solids, the result calculated for an indirect ISE method may be inaccurate. Many illnesses, such as diabetes, liver and kidney disorders, and alcoholism, can raise protein and lipid concentrations in the blood, causing hyperproteinemia and hyperlipidemia, respectively. These conditions do not impact direct ISE, but indirect ISE results depend on the content of solids in a sample because of the dilution step. When the volume of plasma solids increases this can lead to falsely low indirect ISE values for serum electrolytes, especially sodium-a phenomenon known as pseudohyponatremia.

Embodiments of the present invention may therefore support reliable ISE-Methods (namely direct ISE), i.e. enable ISE reading with no systematic, respectively very limited risk of clinical misclassification (i.e. pseudonormonatremia, pseudohypernatremia), while using a minimal amount of patient sample and enabling short cycle times.

According to an embodiment of the present invention, the sample input cup comprises a sample input aperture at a top portion allowing insertion of a pipette or a capillary or a hollow needle, and/or wherein the sample input cup is substantially rotationally symmetric with respect to a longitudinal axis of the sample input cup.

Thereby, the liquid sample may be filled into the sample input cup from above using conventionally used sample containers such as a pipette, a capillary or a hollow needle. The diameter or the extent of the sample input aperture may be defined or selected as required and may range for example between for example 3 mm and 10 mm. Other values are possible. The sample input aperture may for example have a circular shape. The sample input aperture may be the widest portion of the sample input cup. The diameter or extent (in a cross sectional view) of the sample input cup may decrease from top to bottom. When the sample input cup is substantially rotational symmetric (for example having substantially a cylinder symmetry), manufacturing may be simplified, and also filling the liquid sample from a sample supply equipment having substantially circular cross section may be enabled in an easy manner.

In particular, the entire sample input cup may be integrally formed with the reception space wall section and further including for example material delimiting a reception space output conduit which may lead to a reception space output port.

According to an embodiment of the present invention, the sample input cup is funnel shaped and/or bell shaped and/or has a continuous narrowing and/or continuous widening having an apex at a bottom portion, and/or wherein an extent of a cross section of the sample input region perpendicular to the longitudinal axis decreases from the top portion to the bottom portion of the sample input cup, the sample input region being delimited by an inner face of the sample input cup, and/or wherein the extent of a cross section of the sample input region close to or at the bottom portion of the cup is smaller than a diameter of a water drop having volume between 10 µl and 50 µl, such that in particular water wets the inner face of the sample input cup at the bottom portion, and/or wherein a diameter of the sample input region or sample input cup inner face increases by a factor of greater than two from bottom to top of the sample input cup.

When the sample input cup is formed to have an apex at a bottom portion, the sample input cup may support measurement of a small amount of sample which may be present in a bottom region of the sample input cup. In particular, only a very small surface area of an inner face of the sample input cup at a bottom portion may be wetted by the liquid sample, to restrict possible contamination. In particular, a sample having a volume between for example 15 µl and 40 µl may be measured using the arrangement. Thereby, in particular, direct ISE may be supported by embodiments of the present invention. The diameter or extent of the cross section of the sample input region or the inner face of the sample input cup may correspond to a diameter of an output aperture of the sample input cup, as explained below.

The cross sectional size or diameter of the inner face of the sample input cup (or the sample input region) may taper in a continuous manner or stepwise manner from the top portion to the bottom portion of the sample input cup. The tapering of the cross sectional extent or diameter may be such that the inner face may for example be concave at least in a bottom portion, in particular in a second zone as will be explained below.

According to an embodiment of the present invention, the sample input cup comprises at least one side inlet and/or at least one side outlet, in particular including a connector tube, having its longitudinal direction running in a direction including an angle between 30° and 60°, in particular 40° and 50°, with the longitudinal direction of the sample input cup, in particular allowing to provide a sample manifold and/or inlet of working solution and/or inlet of calibration solution and/or inlet of washing solution and/or outlet of waste solution or overflow liquid.

Also the side inlet and/or the side outlet(s) may be integrally formed and comprised in the second portion of the arrangement. The side inlet(s) and/or side outlet(s) may protrude inclined from an external face of a wall having the inner face for delimiting the sample input region. Thereby, wash solution and/or calibration solution may be supplied into the sample input region from external reservoirs, as required for preparation of a measurement, calibration of the measurement apparatus or washing of components of the measurement apparatus. The side outlet may for example be oriented to protrude substantially perpendicular to a longitudinal axis of the sample input cup. The side outlet may for example serve as an overflow outlet at a top portion of the sample input cup.

The side inlet and/or side outlet may have a connecting portion for allowing the connection of a (flexible) tubing. For example, a flexible tubing may be slid above the connecting portion for allowing to convey any calibration liquid or washing liquid via the side inlet to the sample input region. When a liquid is guided via the side inlet into the sample input region, it is discharged from the side inlet in a direction downwards and running along the inner face of the sample input cup, thereby effectively wetting the inner face below the side inlet or liquid communication opening between the side inlet and the sample input cup. Thereby, reliable cleaning of the inner face of the sample input cup may be enabled. If more than one side inlet is present, the side inlets may be inclined at same or different angles relative to the longitudinal axis of the sample input cup or a vertical direction. Inclination angles may be selected or adjusted, such that a desired spiral flow of any solution or liquid introduced via the side inlet is achieved.

According to an embodiment of the present invention, at least one side inlet is oriented and positioned relative to the sample input cup such that liquid supplied by the side inlet discharges substantially tangentially to the inner face of the sample input cup into the sample input cup; and/or wherein the longitudinal direction of the at least one side inlet is parallel to a tangent at the inner face of the sample input cup and the side inlet is positioned such that liquid supplied by the side inlet into the sample input cup wets/streams at a portion of the inner face of the sample input cup immediately adjacent to a liquid communication opening between the side inlet and sample input cup; and/or wherein a liquid communication opening between the side inlet and sample input cup is above a liquid level of a reference amount of liquid filled into the sample input cup.

The side inlet and/or side outlet may comprise a substantially cylindrical lumen, having for example a circular cross sectional shape. When the liquid discharges substantially tangentially to the inner face of the sample input cup, the inner face of the sample input cup may effectively and thoroughly be cleaned by the supplied liquid. Furthermore, a continuous flow may be achieved and spill over may be avoided. The side inlet may be in fluid communication with the sample input region via the liquid communication opening. The liquid communication opening may be above a level of liquid which is reached when for example a sample to be measured is filled into the sample input cup. Thereby, the side inlets may not interfere with any sample liquid. The sample liquid when filled into the sample input cup may not wet the liquid communication opening, thereby reducing contamination.

According to an embodiment of the present invention, the at least one side inlet comprises a first side inlet and a second side inlet arranged at different axial positions of the sample input cup, and in particular substantially at laterally opposite sides of the sample input cup, wherein the first side inlet and a second side inlet in particular allow introduction of liquid into the sample input region in form of a spiral shaped stream at an inner surface of the sample input cup preventing cross-contamination and/or carry over and/or spill over and/or allowing cleaning or purging the inner face of the sample input cup, and/or wherein the side outlet includes an overflow outlet arranged axially above the first side inlet and second side inlet.

The first side inlet and the second side inlet are arranged at different vertical levels of the sample input cup, for example in order to avoid interference or spill over of liquid which is conveyed in one of the side inlets into the other of the side inlets. Embodiments may also provide a first side inlet and a second side inlet not at laterally opposite sides, but circumferentially closer to each other. A spiral shaped stream at the inside surface of the sample input cup may be present immediately below the liquid communication opening, but does not need to necessarily continue until the bottom portion of the sample input cup. Instead, the liquid or solution introduced by one of the side inlets may spread across at least portions of the inner face of the sample input cup which is arranged below the respective liquid communication opening of the considered side inlet. Thereby, thorough and effective cleaning of the inner face of the sample input cup may be achieved.

The overflow outlet may be vertically arranged above any of the side inlets, in particular the first side inlet and the second side inlet. In particular, the overflow outlet may be arranged in an upper portion or top portion of the sample input cup where the cross sectional extent is greater than the cross sectional extent at the vertical or axial position where any of the side inlets is positioned. Both, by widening the extent or diameter of the sample input cup in the top portion and further by providing the side outlet configured as an overflow outlet, spill of any liquid from the inside of the sample input cup to the outside may be avoided.

According to an embodiment of the present invention, the sample input cup comprises an output aperture at a bottom portion, in particular in a central region of the bottom portion, wherein the second portion comprises material below the output aperture configured to circumferentially delimit a, in particular straight, input conduit, in particular substantially oriented along a longitudinal axis of the sample input cup, providing fluid communication between the sample input region and the reception space, wherein an outer surface of material delimiting the input conduit serves as adherent surface at which the first portion is attached, in particular glued.

The output aperture may provide a fluid or liquid communication between the sample input region (provided by the sample input cup) and the reception space, in particular via the input conduit. Herein, the input conduit is understood as an input conduit leading to the reception space. Thereby, the material of the second portion delimiting in particular the input conduit may also be integrally formed. Thus, also the input conduit may not be formed by a separate element or unit, but may be integrally formed as part of the second portion. Thereby, also possible contamination of sample liquid at gaps or spaces between connected elements, as observed in the conventional systems, may be reduced or even avoided.

The sample input conduit may for example be arranged such that a longitudinal central axis of the input conduit coincides with a longitudinal central axis of the sample input cup or the sample input region. Thereby, also manufacturing may be simplified. Furthermore, when the input conduit is substantially straight, flow resistance during conveying any liquid contained within the sample input cup into the reception space (for measurement) may be reduced. Furthermore, loss of sample liquid may be reduced.

When the adherent surface or interface surface of the second portion is provided, connection or coupling to the first portion may be simplified. Connecting or coupling the first portion with the second portion may be liquid tight such that in particular the reception space is liquid tight, when the first portion is coupled with the second portion.

According to an embodiment of the present invention, the first portion and the second portion each comprise respective, in particular plane, interface surface regions provided to form adherent surfaces at which the first portion and the second portion are mounted, in particular glued, together, and/or wherein the interface surface region of the second portion is laterally offset from an input conduit axis of the input conduit towards the first side, in particular due to material delimiting the input conduit at the first side.

The interface surface regions may advantageously be used to couple the first portion with the second portion. The first portion and the second portion may be coupled via one or more different connection means, such as bolts, one or more clamps and the like and may in particular be glued together such that respective interfacing surface regions contact each other via an adhesive. When the interface surface region of the second portion is laterally offset from an input conduit axis of the input conduit towards the first side, the input conduit may advantageously be arranged to run substantially with its central longitudinal axis aligned or coinciding with the longitudinal axis of the sample input cup.

According to an embodiment of the present invention, the sample input cup comprises at least one of the following zones: a first zone, in particular at the bottom portion, comprising a continuously changing diameter, wherein an inner surface of the first zone includes in particular an angle between 10° and 60° with a longitudinal axis of the sample input cup; a second zone, in particular at a middle portion, comprising a substantially constant diameter and having one or more inlets, wherein an inner surface of the second zone in particular includes an angle between 0° and 10° with a longitudinal axis of the sample input cup; a third zone, in particular at a top portion, comprising a continuously changing diameter and/or one or more step wise changes of the diameter, wherein an inner surface of the third zone includes in particular an angle between 40° and 60° with a longitudinal axis of the sample input cup.

The zones may be understood as zones in different vertical regions of the sample input cup. In the first zone, the inner face of the sample input cup may for example have a concave shape, thereby allowing to increase the diameter in the direction vertically upwards. Thus, the angle between a tangent (in a longitudinal section) at the inner surface and the longitudinal axis may change within the first zone. In the second zone, the angle included between the inner face and the longitudinal axis of the sample input cup may substantially be constant and may in particular be around 0°. In the third zone, the diameter or the extent of the input cup may increase for example in a linear manner in dependence of the vertical position. Between the second zone and the third zone, the angle between the inner face of the sample input cup and the longitudinal axis of the sample input cup may abruptly change, for example by between 5° and 20°. Thereby, it is enabled to provide within the third zone a volume which considerably increases along the vertical direction upwards, in order to in particular avoid spill over any liquid contained within the sample input cup.

According to an embodiment of the present invention, the second portion is integrally formed, in particular manufactured by injection molding, further in particular using polycarbonate, polyester, polyamides and blends thereof, and/or the second portion further comprising, in particular downstream the reception space: a reception space output port including a tube connector, and/or wherein the measurement area comprises at least one analyte sensitive area, in particular ion-selective electrode, and/or wherein the arrangement including the sample input cup is configured to support direct ion selective electrode measurements of undiluted sample as input via the sample input cup.

Thereby, manufacturing of the second portion may be simplified and performed using conventionally available techniques. The reception space output port has the tube connector which may for example allow to connect a flexible tubing. Downstream the flexible tubing for example a pump, in particular suction pump or peristaltic pump, may be provided for enabling to convey any liquid contained within the sample input cup into the reception space and further to convey it out of the reception space, for example after measurement. The measurement area may comprise for example one or more ion selective electrodes and/or one or more optodes and/or one or more metabolic sensors and/or one or more amperometric sensors or the like, as required by the intended measurement. Thereby, conventionally intended measurement procedures may be supported and sample amount, as required by the measurement, may be reduced.

According to an embodiment of the present invention, the arrangement further comprises at least one suction pump, connected to the reception space output port; and/or a liquid column separator connected to the suction pump, to receive liquid at an input port and configured to output the liquid via an output port, in particular to a waste compartment, such that a continuous liquid column between the input port and the output port of the liquid column separator is interrupted.

The suction pump may for example be configured as a peristaltic pump. Thereby, conveying any liquid into and out of the reception space may be enabled. The liquid column separator may be configured to avoid any continuous liquid column between regions upstream of the liquid column separator and regions downstream the liquid column separator. Thereby, electromagnetic interference due to electromagnetic signals coupled into a conducting liquid column may be reduced or even avoided. Thereby, reliability of measurement results may be improved. In particular, liquid upstream the liquid column separator may, due to the function of the liquid column separator, electrically be disconnected from any liquid downstream the liquid column separator.

According to an embodiment of the present invention, the liquid column separator comprises at least one, in particular substantially vertically arranged, guiding member having a guiding surface at which incoming liquid is collected and is guided to run downwards, at least one drip off edge at a bottom of the guiding member; a bottom collection region vertically spaced apart from and below the drip off edge, wherein the liquid column separator is in particular formed by an upper member comprising the guiding member and formed by a lower member forming the bottom collection region.

Liquid may enter the liquid column separator via the input port and may be guided across or along the guiding surface. The liquid guided downwards at the guiding surface may then reach the drip off edge and may then drip off from the drip off edge to be collected in the bottom collection region which is separated and (vertically) spaced apart from the drip off edge. The bottom collection region may then be in liquid communication with the output port of the liquid column separator. Thereby, a simple construction is provided, simplifying also the manufacturing.

According to an embodiment of the present invention, for guiding the liquid two parallel and spaced apart, in particular transparent, guiding members (e.g. plates) are provided between which the liquid is allowed to run downwards as a continuous liquid film, and/or wherein an optical measurement is enabled in transmission through the transparent guiding plates and the liquid in between.

Liquid may be guided between the two parallel guiding members. In particular, the liquid may be guided between the two parallel guiding members or guiding surfaces or guiding plates such that no bubbles are present between the guiding plates. Thereby, one or more optical measurements (in visible and/or infrared and/or ultraviolet spectral range) may be enabled. For example, one or more radiation sources such as light sources may be provided and measurement light may be generated and guided through the liquid which runs between the two parallel guiding members. Furthermore, one or more detectors may be provided to measure intensity of transmitted light transmitted through the liquid guided between the two parallel guiding members. Thereby, further analysis of the sample may be enabled.

It should be understood, that features, individually or in any combination, disclosed, described, explained or provided for an arrangement for measuring at least one constituent of a liquid sample, may also, individually or in any combination, be applied or provided for a method of measuring at least one constituent of a liquid sample; or a method of manufacturing an arrangement for measuring at least one constituent of a liquid sample, according to embodiments of the present invention and vice versa.

According to an embodiment of the present invention it is provided a method of measuring at least one constituent of a liquid sample, in particular comprising serum, plasma and/or urine, the method comprising: filling the sample into a sample input region delimited by a sample input cup; partly delimiting a reception space for the sample by a first portion at a first side, wherein the first portion comprises at least one measurement area; partly delimiting the reception space at a second side by a reception space wall section of a second portion coupled to the first portion; wherein the sample input region is in fluid communication with the reception space, wherein the reception space wall section and the sample input cup are integrally formed; the method in particular further comprising: guiding the sample into the reception space; and measuring the sample in contact with the measurement area.

Furthermore, according to an embodiment of the present invention it is provided a method of manufacturing an arrangement for measuring at least one constituent of a liquid sample, the method comprising: manufacturing a first portion comprising at least one measurement area; manufacturing a second portion comprising a reception space wall section and a sample input cup; coupling the second portion to the first portion such that the reception space is delimited by the first portion at a first side, and such that the reception space is partly delimited by the reception space wall section at a second side; wherein the sample input cup is configured to delimit a sample input region into which the sample is fillable, wherein the sample input region is in fluid communication with the reception space, wherein the reception space wall section and at least a portion of the sample input cup are integrally formed.

The sample input cup may according to an embodiment, in particular entirely, protrude beyond a top of the first portion. The first portion may extend below the output aperture but not above the output aperture. The reception space output port may include a tube connector arranged vertically at a higher level than the measurement area.

According to an embodiment of the present invention, the first portion comprises a polymer formed as a printed circuit board. The polymer may effectively adhere to the second portion when a conventionally available adhesive is used. Further, manufacturing the first portion may thereby be simplified. In particular, a conventionally available printed circuit board material may be used and conventionally available techniques may be applied to form copper conductive traces and/or sensor areas and/or other electronic elements onto the polymer according to the requirements of the measurement system and the requirements of the heating/temperature detection system. The printed circuit board may be layered.

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

### Brief Description of the Drawing

Embodiments of the present invention are now described with reference to the accompanying drawings. The invention is not restricted to the illustrated or described embodiments.
Fig. 1 schematically illustrates an arrangement for measuring at least one constituent of a liquid sample according to an embodiment of the present invention;
Figs. 2, 3, 4, 5 schematically illustrate a second portion of an arrangement for measuring a constituent of a liquid sample according to an embodiment of the present invention, in a side view, in a sectional top view, in a cross sectional side view and in a cross sectional longitudinal view, respectively;
Fig. 6 schematically illustrates a second portion of an arrangement for measuring a constituent of a liquid sample according to an embodiment of the present invention in a sectional side view;
Fig. 7 schematically illustrates an arrangement for measuring at least one constituent of a liquid sample according to an embodiment of the present invention in a cross sectional longitudinal view; and
Figs. 8, 9, 10 illustrate a liquid column separator according to an embodiment of the present invention in different sectional views.

### Detailed Description

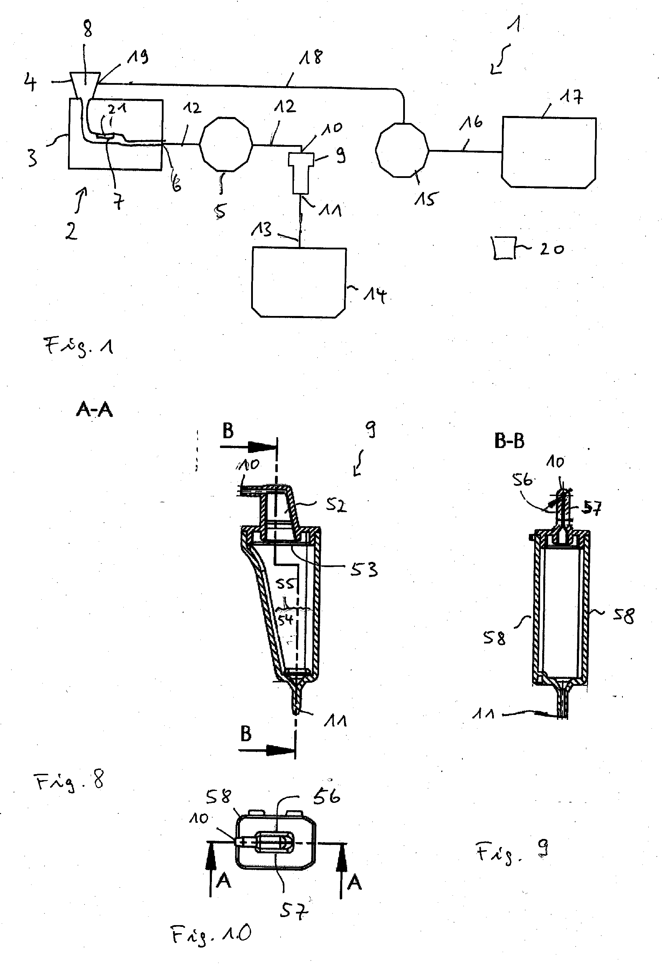

The arrangement 1 for measuring at least one constituent of a liquid sample according to an embodiment of the present invention illustrated in **Fig. 1** in a schematic manner includes a measurement cell 2 which includes a first portion and a second portion (which will be described in detail below), wherein the first portion and the second portion is collectively labelled with reference sign 3. The second portion comprises a sample input cup 4. The first portion and the second portion (collectively labelled with reference sign 3) delimit a reception space 7 which is in fluid communication with a sample input region 8 provided in the interior of the sample input cup 4. The reception space 7 is further in fluid communication with the reception space output port 6.

The arrangement 1, which may also be referred to as a measurement apparatus, further comprises a fluid transportation system 5 which is connected to a reception space output port 6 of the measurement cell 2. The fluid transportation system may be configured as a peristaltic pump. The fluid transportation system 5 is provided for aspiration and positioning of a liquid sample (for example patient sample) and/or working solution and/or calibrant solution, in fluid connection on a first end with the reception space output port 6 of the measurement cell 3 and on the second end in fluid communication with a liquid column separator 9 (also referred to as hose line separator) which will be described in detail below. Thus, the liquid column separator 9 is connected to the transportation system 5 or in particular the suction pump to receive liquid at an input port 10 and is configured to output the liquid via an output port 11 such that a continuous liquid column between the input port 10 and the output port 11 of the liquid column separator is interrupted. Thereby, the hose line separator or liquid column separator 9 electrically disconnects the measurement cell 3 from a closed column of conductive liquids and/or conductive coatings on the surface of tubings downstream from the liquid column separator 9, in particular the waste tubing 13, to avoid electrical interferences. The liquid column separator 9 is at the input port 10 in fluid communication with the fluid transportation system 5 and is connected at the output port 11 in fluid communication with the waste tubing 13. The waste tubing 13 connects the liquid column separator 9 and a waste compartment 14 which collects waste liquid.

The arrangement 1 further comprises a further fluid or liquid transportation system 15 provided for working solutions and calibrant solutions. The further transportation system 15 may typically comprise several peristaltic pumps in fluid connection (via tubing 16) with fluid compartments 17 holding working solutions and/or calibrants and on the other hand via tubing 18 to specific inlets 19 (in particular one or more inlets 19) leading to the interior 8 of the sample input cup 4. In the compartments 17, one or more containers for working solutions and/or calibrant solutions may be provided.

The arrangement 1 further comprises a control unit 20 which is communicatively connected to one or more of the other elements of the arrangement 1, in particular with the transportation systems 5 and/or 15 and with circuitry or electrodes of a measurement area 21 provided such that at least one sensitive area of the measurement area 21 is accessible from the reception space 7. The control unit 20 may be configured to acquire measurement data and may be configured to perform signal processing and/or system control and/or power supply and/or data exchange with an exterior equipment. In particular, the control unit 20 may be in electrical connection with the measurement cell 3, in particular the measurement area 21, and may be in electrical connection with one or more of the solution or liquid transport systems 5, 15.

The arrangement 1 is configured to support direct ISE methods and/or indirect ISE methods, in order to determine a plurality of ion activities of a liquid sample. The highly integrated measurement cell comprises a first portion (described below in detail) including all analyte electrodes and/or reference electrodes and/or sensors for fluidic control, in particular fluid and bubble detection and/or elements for thermostatic control and comprises a fluid channel including the reception space 7. The sample input cup 4 serves as sample compartment and/or sample manifold having specific inlets 19 for working solutions and/or calibrant.

The first portion 24 may be coupled to the second portion 22 by gluing at respective interface portions. The first portion 24 may be configured as a flat plate, in particular configured as a printed circuit board.

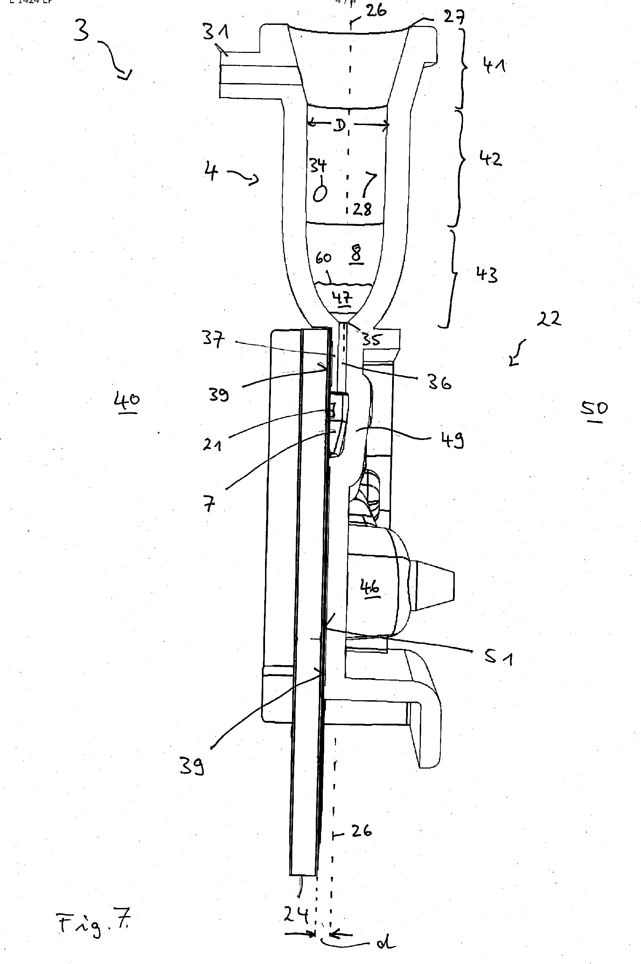

The sample input cup 4 delimits the sample input region 8 into which a sample or a calibration solution or a washing solution, for example may be filled. The second portion 22 comprises a reception space wall section (49, illustrated e.g. in Fig. 7) which partly delimits the reception space 7 illustrated in Fig. 1. The sample input cup 4 is integrally formed with the reception space wall section. The measurement cell 3 may comprise a housing and may be designed as a consumable and is insertable and retractable from other components of a measuring apparatus .

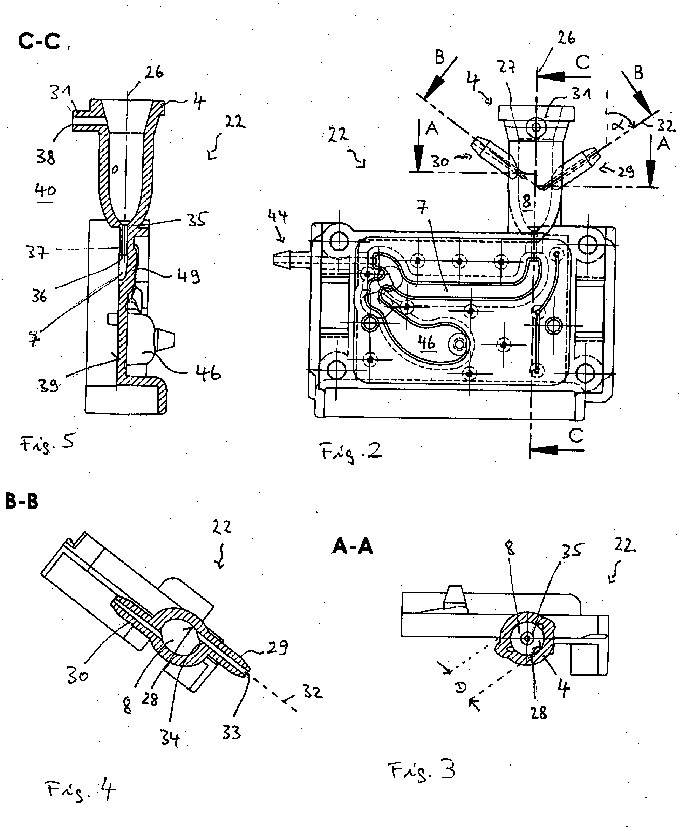

**Fig. 2** schematically illustrates the second portion 22 in a schematic side view. Therein, a longitudinal axis 26 of the sample input cup 4 is oriented vertically for performing a measurement. The sample input cup 4 comprises a sample input aperture 27 at a top portion allowing insertion of a pipette or a capillary or a hollow needle. In the embodiment illustrated in Figs. 2, 3, 4, 5, the sample input cup 4 is substantially rotationally symmetric with respect to the longitudinal axis 26 of the sample input cup 4. As can be appreciated for example from Figs. 2, 5, the sample input cup is bell-shaped and has an apex at a bottom portion.

The sample input cup 4 comprises a first side inlet 29 and a second side inlet 30 which are arranged at different vertical positions (and thus axial positions of the sample input cup 4). Further, these side inlets 29, 30 are arranged laterally at opposite sides of the sample input cup 4. The sample input cup 4 further comprises a side outlet 31 which is vertically above the first inlet 29 and the second inlet 30. As can be taken from Fig. 2, a longitudinal axis 32 of the first inlet 29 is inclined by an angle α with respect to the vertical direction 26. The angle α may be within a range of 30° to 60°, for example. The second inlet 30 may be inclined by a similar angle, but in the opposite direction.

**Fig. 3** schematically illustrates the first portion 22 in a cross sectional view taken along the line labelled with A-A in Fig. 2. Therein, the sample input region 8 is delimited by an inner face 28 of the sample input cup 4. The cross sectional size or the diameter of the inner face 28 of the sample input cup 4 increases from the bottom portion to the top portion of the sample input cup. The diameter D of the sample input region 8 (or the inner face 28 of the sample input cup 4) is indicated in Fig. 3.

**Fig. 4** schematically illustrates a cross sectional view of the second portion 22 as taken along the lines labelled with B-B in Fig. 2.
The first inlet 29 comprises a lumen 33 shaped as a cylinder and being arranged to run tangentially to the inner face 28. Thus, liquid supplied via the side inlet 29 into the sample input region 8 will discharge substantially tangentially to the inner face 28 of the sample input cup 4 into the sample input cup or into the sample input region 8. The lumen 33 of the side inlet 29 is in fluid communication with the sample input region 8 via a liquid communication opening 34 at the end of the inlet 29. When liquid is supplied via the first inlet 29 into the sample input region 8, the liquid will wet a portion of the inner face 28 close to the liquid communication opening 34.

As can be appreciated from Fig. 3 and **Fig. 5****,** the sample input cup 4 comprises an output aperture 35 at the bottom portion, for outputting liquid contained within the sample input region 8 towards the reception space 7 which is also illustrated in Fig. 2. Between the sample input region 8 and the reception space 7, an input conduit 36 is arranged which is in particular oriented and arranged along the longitudinal axis 26 of the sample input cup 4. The input conduit 36 is in the illustrated embodiment a straight conduit having a cylindrical cross section. The reception space 7 is arranged below the input conduit. The input conduit 36 is delimited by material 37 comprised in the second portion 22.

The material 37 delimiting the input conduit 36 is illustrated in Fig. 5 which illustrates a cross sectional view of the second portion 22 when taken at the sectional line labelled C-C in Fig. 2. From Fig. 5 it can be appreciated that the side outlet 31 provides a lumen 38 for transporting liquid, wherein the lumen 38 runs perpendicular to the longitudinal direction 26 of the sample input cup. In Fig. 5, also interface surface regions 39 provided at the second portion 22 are illustrated. These interface surface regions 39 provide adherent surfaces to which the first portion 24 (illustrated in Fig. 7) may be glued. As can be appreciated from Fig. 5, the interface surface region 39 of the second portion 22 is laterally offset by an amount d (indicated in Fig. 7) from the input conduit axis 26 towards the first side 40, in particular due to the material 37 delimiting the input conduit 36 at the first side 40.

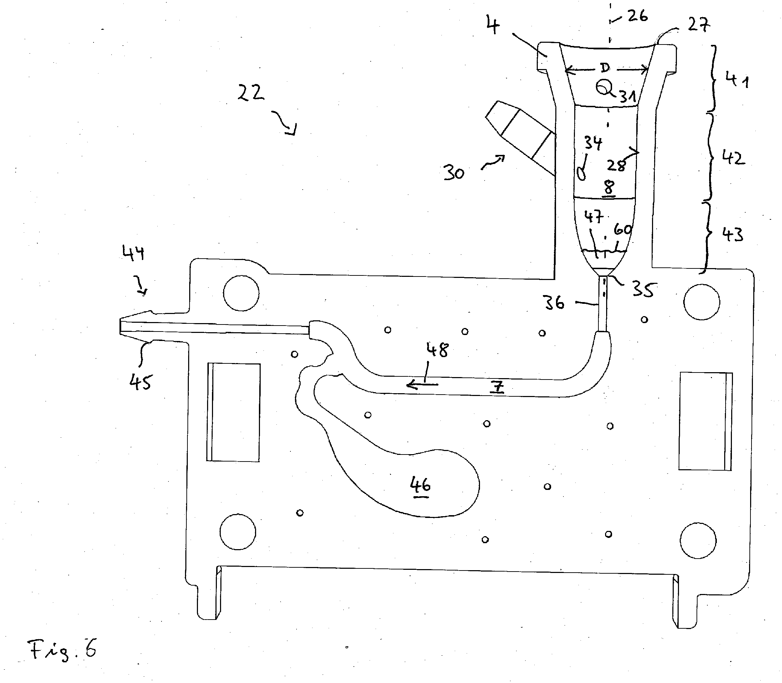

**Fig. 6** schematically illustrates the first portion 22 in a sectional side view. Therein, the second side inlet 30 opens into the sample input region8 provided by the sample input cup 4 via the liquid communication opening 34. The sample input aperture 27 of the sample input cup 4 is arranged within a third zone 41, in particular at a top portion. In the third zone 41, the diameter D of the input region 8 increases linearly for increasing vertical position along the longitudinal axis 26.

The inlets, in particular the first and second inlet 30, may be provided in a second zone 42 of the sample input cup 4, being vertically below the third zone 41. Within this zone 42, the diameter D may substantially be constant. The sample input cup 4 further comprises a first zone 43 at a bottom region which may also comprise the output aperture 35 leading to the input conduit 36. In the first zone, the diameter D may continuously change along the vertical direction. According to embodiments of the present invention, the sample input cup may comprise 1 or more of all these zones in any combination. For example, the sample input cup 4 does not necessarily comprise a zone having substantially constant diameter. The inlet 30 or further inlets may alternatively be connected or provided within the first zone and/or the third zone and the outlet.

Downstream the reception space 7 delimited by material of the second portion 22, a reception space output port 44 is provided having a tube connector 45 over which for example a flexible tubing may be drawn. The second portion 22 also partly delimits a region 46 branching off from the fluid connection path from the reception space 7 towards the reception space output port 44.
Region 46 (an optional feature) forms a reservoir which acts as a compartment for the inner electrolyte of a reference electrode necessary for potentiometric measurement.

For loading, a liquid sample 47 (e.g. having filling level 60 below side inlets) contained within the sample input cup 4 into the reception space 7, the liquid is conveyed according to the flow direction 48 illustrated in Fig. 6. The reception space output port 44 may be connected for example via a pump 5 and a liquid column separator 9 towards a waste compartment 14 as illustrated in Fig. 1.

**Fig. 7** schematically illustrates a measurement cell 3 in a cross sectional side view corresponding to the viewing direction of Fig. 5. The measurement cell 3 comprises a second portion 22 (at a second side 50) and a first portion 24 (at a first side 40). The second portion 22 includes a reception space wall section 49 which partly delimits the reception space 7. The first portion 24 comprises at least one measurement area 21 and delimits the reception space 7 at the first side 40. The reception space wall section 49 of the second portion 22 delimits the reception space 7 at the second side 50. As can be seen in Fig. 7, the first portion 24 is designed or constructed as a flat plate, in particular a printed circuit board. Via the interface surface region 39 of the second portion 22 and an interface surface region 51 of the first portion 24, the first portion 24 and the second portion 22 are coupled to each other. The interface surface regions 39, 51 are in particular plane regions. The material 37 below the output aperture 35 of the second portion 22 circumferentially delimits the input conduit 36 providing a fluid communication between the sample input region 8 and the reception space 7. The measurement cell 3 is insertable into a reception opening or reception slot of a measuring apparatus and may be replaced on a regular basis as a consumable.

**Figs. 8, 9, 10** schematically illustrate in sectional views a liquid column separator 9 according to an embodiment of the present invention, which may for example be comprised in the arrangement 1 illustrated in Fig. 1. Fig. 8 illustrates a sectional side view as viewed along the section line labelled A-A in Fig. 11; Fig. 9 illustrates a sectional view taken along the section line labelled B-B illustrated in Fig. 8; Fig. 10 illustrates a top view of the liquid column separator. The liquid column separator 9 comprises an input port 10 via which liquid is input into the liquid column separator 9. The liquid column separator 9 is configured to output the liquid via an output port 11 such that a continuous liquid column between the input port 10 and the output port 11 is interrupted. Therefore, the liquid column separator 9 comprises a guiding member having a guiding surface 52 at which the incoming liquid is collected and guided to run downwards, i.e. downwards in the vertical direction. The liquid column separator or in particular the guiding surface 52 comprises a drip off edge 53 at the bottom of the guiding member 52. The liquid drops off the drip off edge 53 and is collected in a bottom collection region 54. The bottom collection region 54 may be filled up to a level 55 which is below the drip off edge 53, such that the liquid within the bottom collection region 54 is electrically disconnected from any liquid at or upstream the drip off edge 53.

The guiding member 52 is formed by two parallel and spaced apart guiding plates 56, 57 as is illustrated in Fig. 10. The liquid is guided to stream between the guiding plates 56, 57. Not illustrated optical light source(s) and detector(s) may be provided for allowing one or more optical measurements for additionally analyzing the sample. The liquid column separator 9 may be arranged between the transportation system 5 and the waste compartment 14 as illustrated in Fig. 1. The bottom collection region is delimited by casing walls 58.

According to embodiments of the present invention, the following features may apply to any of the aforementioned embodiments:
The fluid channel of the measurement cell may be provided with a rotationally symmetrically widening (as an example of a sample input cup) on a first end of the measuring channel which may serve as a sample compartment and/or sample manifold having specific inlets for a working solution and/or calibrant. The diameter of the inner face of the widening may increase at least one time from bottom to top.

The lower portion of the widening may be formed in a manner that aliquots of the test solution touch the inside of the rotationally symmetrically inner face, as a result of which drops that form on the tip of the sample transfer system, in particular a pipette, are fully captured by the inner face and a complete filling of the channel is guaranteed. This may enable to dispense very small volumes and to measure very small volumes of sample. The upper portion of the widening may be formed in a manner that at least one supply line protrudes inclined tangentially into the rotationally symmetrically surface, whereas the distance between the supply line and the area with the minimum radius of the surface being selected so that no closed column of liquid is formed without active movement of the liquid. This may enable an electrical decoupling of the sensor area and the supply lines of the measurement cell which subsequently leads to increased interference immunity.

The inclined tangential coupling of the supply line may create a downswing spiral flow which may lead to a better cleaning of the inner face in contact with the test solution, in particular patient sample and calibrant and/or working solution. The sample input cup may comprise one or more supply lines. In case there are plural supply lines, the position of any of these supply lines or inlet ports may be selected so that both the distance between the lines and the resulting direction of the rotation of the spiral flow prevent cross contamination. The upper portion of the widening (for example supply sample input cup) may be formed in a manner to prevent overflow and/or mitigate the effects of an overflow and/or leakage of test solution, in particular patient sample and/or calibrant and/or working solution. This may be achieved by an additional increase of the diameter of the inner face of the widening and/or by having a drain line protruding the inner face, through which the liquid can be drained off in a targeted manner.

The second end of the flow channel may be in fluid communication with a fluid transportation system which may be in immediate vicinity of the second end of the channel.

The measurement cell, in particular the flow channel, may be integrally formed, in particular manufactured by injection moulding, further in particular using polycarbonate, polyester, polyamides and blends thereof. This may enable the integration of a sample compartment and/or sample manifold having specific inlets for working solution and/or calibrant into a consumable which can be replaced at a regular base without having economical drawbacks. Further, this may enable that long-term effects such as overlay coating and/or surface modification coating of biological samples, in particular biofouling, can be avoided by cyclical replacement and does not require extensive cleaning and/or other maintenance activities at a regular base, yielding into higher uptime of the system and minimized user interaction.

The fluid transportation system (for example illustrated in Fig. 1 labelled with reference sign 5) is in particular a peristaltic pump in immediate vicinity of the second end of the channel, whereas the pump may be used to aspirate and position the test solution, in particular patient sample and/or calibrant and/or working solution when active. The pump 5 may be used as a valve when inactive. Thereby, no unwanted negative pressure may be developed during the high flow rate portions of the cycle, enabling small amounts of the test solution to be aspirated and/or positioned.

The liquid column separator 5 or also called hose line separator 5 may be composed of at least two hollow bodies which may be connected in such a way that the liquid drips of, thereby interrupting a closed column of liquid, whereas the diameter of the inner face of the lower body exceeds the inner face of the upper body and the delimitation of the inner surface of the upper body penetrates the inner volume of the lower body. Thereby, an edge (for example edge 53 illustrated in Fig. 8) is formed at which the liquid is forced to drip off. This may enable the electrical decoupling of the hose lines, in particular waste tubing and measurement cell 3 which subsequently leads to increased interference immunity.

The body of the liquid column separator may be shaped in such a way that two plane parallel transparent surfaces (for example 56, 57 illustrated in Fig. 10) are provided which are at a defined distance from one another so that a kind of cuvette is formed for the optical determination of sample components. The liquid column separator 9 may be integrally formed, in particular manufactured by injection moulding, further in particular using polycarbonate, polyester, polyamides and blends thereof.

The measurement cell may for example be replaced on a regular base or after a predefined number of measurements have been performed.

It should be noted that the term "comprising" does not exclude other elements or steps and the use of articles "a" or "an" does not exclude a plurality. Also elements described in association with different embodiments may be combined. It should also be noted that reference signs in the claims should not be construed as limiting the scope of the claims. Further, spatially relative terms, such as "front" and "back", "above" and "below", "left" and "right", et cetera are used to describe an element's relationship to another element(s) as illustrated in the Figures. Thus, the spatially relative terms may apply to orientations in use which differ from the orientation depicted in the Figures. Obviously all such spatially relative terms refer to the orientation shown in the Figures only for ease of description and are not necessarily limiting as an apparatus according to an embodiment of the invention can assume orientations different than those illustrated in the Figures when in use.

## Claims

1. Arrangement (1) for measuring at least one constituent of a liquid sample (47), in particular comprising serum, plasma and/or urine, the arrangement comprising:
a first portion (24) adapted to partly delimit a reception space (7) for the sample at a first side (40) and comprising at least one measurement area (21);
a second portion (22) comprising:
a reception space wall section (49), the second portion being coupled to the first portion (24) such that the reception space wall section (49) partly delimits the reception space (7) at a second side (50); and
a sample input cup (4) delimiting a sample input region (8) into which the sample is fillable,
wherein the sample input region (8) is in fluid communication with the reception space (7),
wherein the reception space wall section (49) and at least a portion of the sample input cup (4) are integrally formed.

2. Arrangement according to the preceding claim,
wherein the sample input cup (4) comprises a sample input aperture (27) at a top portion allowing insertion of a pipette or a capillary or a hollow needle, and/or
wherein the sample input cup (4) is substantially rotationally symmetric with respect to a longitudinal axis (26) of the sample input cup.

3. Arrangement according to one of the preceding claims,
wherein the sample input cup (4) is funnel shaped and/or bell shaped and/or has a continuous narrowing and/or continuous widening having an apex at a bottom portion, and/or
wherein an extent (D) of a cross section of the sample input region (8) perpendicular to the longitudinal axis (26) decreases from the top portion to the bottom portion of the sample input cup, the sample input region being delimited by an inner face (28) of the sample input cup,
and/or
wherein the extent (D) of a cross section of the sample input region close to or at the bottom portion of the cup is smaller than a diameter of a water drop having volume between 10 µl and 50 µl, such that in particular water wets the inner face of the sample input cup at the bottom portion, and/or
wherein a diameter (D) of the sample input region (8) or sample input cup inner face (28) increases by a factor of greater than two from bottom to top of the sample input cup.

4. Arrangement according to one of the preceding claims,
wherein the sample input cup (4) comprises at least one side inlet (29, 30) and/or at least one side outlet (31), in particular including a connector tube, having its longitudinal direction (32) running in a direction including an angle (a) between 30° and 60°, in particular 40° and 50°, with the longitudinal direction (26) of the sample input cup (4),
in particular allowing to provide a sample manifold and/or inlet of working solution and/or inlet of calibration solution and/or inlet of washing solution and/or outlet of waste solution or overflow liquid.

5. Arrangement according to one of the preceding claims,
wherein the at least one side inlet (29, 30) is oriented and positioned relative to the sample input cup such that liquid supplied by the side inlet discharges substantially tangentially to the inner face (28) of the sample input cup (4) into the sample input cup; and/or
wherein the longitudinal direction (32) of the at least one side inlet (29) is parallel to a tangential at the inner face (28) of the sample input cup (4) and/or the side inlet (29, 30) is positioned such that liquid supplied by the side inlet into the sample input cup wets/streams at a portion of the inner face of the sample input cup immediately adjacent to a liquid communication opening between the side inlet and sample input cup; and/or
wherein a liquid communication opening (34) between the side inlet (30) and sample input cup (4) is above a liquid level (60) of a reference amount of liquid filled into the sample input cup (4).

6. Arrangement according to one of the preceding claims,
wherein the at least one side inlet comprises a first side inlet (29) and a second side inlet (30) arranged at different axial positions of the sample input cup, and in particular substantially at laterally opposite sides of the sample input cup (4),
wherein the first side inlet and a second side inlet in particular allow introduction of liquid into the sample input region in form of a spiral shaped stream at an inside surface of the sample input cup preventing cross-contamination and/or carry over and/or spill over and/or allowing cleaning or purging the inner face of the sample input cup, and/or
wherein the side outlet (31) includes an overflow outlet arranged axially above the first side inlet (29) and second side inlet (30).

7. Arrangement according to one of the preceding claims,
wherein the sample input cup (4) comprises an output aperture (35) at a bottom portion, in particular in a central region of the bottom portion,
wherein the second portion (22) comprises material (37) below the output aperture (35) configured to circumferentially delimit a, in particular straight, input conduit (36), in particular substantially oriented along a longitudinal axis (26) of the sample input cup, providing fluid communication between the sample input region (8) and the reception space (7),
wherein an outer surface (39) of material delimiting the input conduit serves as adherent surface at which the first portion (24) is attached, in particular glued.

8. Arrangement according to one of the preceding claims,
wherein the first portion (24) and the second portion (22) each comprise respective, in particular plane, interface surface regions (39, 51) provided to form adherent surfaces at which the first portion and the second portion are mounted, in particular glued, together, and/or
wherein the interface surface region (39) of the second portion is laterally offset from an input conduit axis (26) of the input conduit (36) towards the first side, in particular due to material (37) delimiting the input conduit at the first side.

9. Arrangement according to one of the preceding claims, wherein the sample input cup comprises at least one of the following zones:
a first zone (43), in particular at the bottom portion, comprising a continuously changing diameter, wherein an inner surface of the first zone includes in particular an angle between 10° and 60° with a longitudinal axis of the sample input cup;
a second zone (42), in particular at a middle portion, comprising a substantially constant diameter and having one or more inlets, wherein an inner surface of the second zone in particular includes an angle between 0° and 10° with a longitudinal axis of the sample input cup;
a third zone (41), in particular at a top portion, comprising a continuously changing diameter or one or more step wise changes of the diameter, wherein an inner surface of the third zone includes in particular an angle between 40° and 60° with a longitudinal axis of the sample input cup.

10. Arrangement according to one of the preceding claims,
wherein the second portion (22) is integrally formed, in particular manufactured by injection molding, further in particular using polycarbonate, polyester, polyamides and blends thereof, and/or
the second portion further comprising, in particular downstream the reception space:
a reception space output port (44) including a tube connector (45), and/or
wherein the measurement area (21) comprises at least one analyte sensitive area, in particular ion-selective electrode, and/or
wherein the arrangement including the sample input cup (4) is configured to support direct ion selective electrode measurements of undiluted sample as input via the sample input cup.

11. Arrangement according to the preceding claim, further comprising at least one of:
at least one suction pump (5), connected to the reception space output port;
a liquid column separator (9) connected to the suction pump, to receive liquid at an input port (10) and configured to output the liquid via an output port (11), in particular to a waste compartment (14), such that a continuous liquid column between the input port and the output port of the liquid column separator is interrupted.

12. Arrangement according to one of the preceding claims, the liquid column separator comprising:
at least one, in particular substantially vertically arranged, guiding member (52) having a guiding surface at which incoming liquid is collected and is guided to run downwards,
at least one drip off edge (53) at a bottom of the guiding member;
a bottom collection region (54) vertically spaced apart from and below the drip off edge,
wherein the liquid column separator is in particular formed by an upper member comprising the guiding member and formed by a lower member forming the bottom collection region.

13. Arrangement according to one of the preceding claims,
wherein for guiding the liquid two parallel and spaced apart, in particular transparent, guiding plates (56, 57) are provided between which the liquid is allowed to run downwards as a continuous liquid film, and/or
wherein an optical measurement is enabled in transmission through the transparent guiding plates (56, 57) and the liquid in between.

14. Method of measuring at least one constituent of a liquid sample (47), in particular comprising serum, plasma and/or urine, the method comprising:
filling the sample into a sample input region (8) delimited by a sample input cup (4);
partly delimiting a reception space (7) for the sample by a first portion (24) at a first side (40), wherein the first portion comprises at least one measurement area (21);
partly delimiting the reception space (7) at a second side (50) by a reception space wall section (49) of a second portion (22) coupled to the first portion (24);
wherein the sample input region (8) is in fluid communication with the reception space (7),
wherein the reception space wall section and the sample input cup are integrally formed;
the method in particular further comprising:
guiding the sample into the reception space (7); and
measuring the sample (47) in contact with the measurement area (21).

15. Method of manufacturing an arrangement (1) for measuring at least one constituent of a liquid sample, the method comprising:
manufacturing a first portion (24) comprising at least one measurement area;
manufacturing a second portion (22) comprising a reception space wall section (49) and a sample input cup (4);
coupling the second portion (22) to the first portion (24)
such that the reception space (7) is delimited by the first portion (24) at a first side (40), and
such that the reception space (7) is partly delimited by the reception space wall section (49) at a second side (50);
wherein the sample input cup (4) is configured to delimit a sample input region (8) into which the sample is fillable,
wherein the sample input region (8) is in fluid communication with the reception space (7),
wherein the reception space wall section (47) and at least a portion of the sample input cup (4) are integrally formed.

## Amended claims

### Amended claims in accordance with Rule 137(2) EPC.

1. Arrangement (1) for measuring at least one constituent of a liquid sample (47), in particular comprising serum, plasma and/or urine, the arrangement comprising:
a first portion (24) adapted to partly delimit a reception space (7) for the sample at a first side (40) and comprising at least one measurement area (21);
a second portion (22) comprising:
a reception space wall section (49), the second portion being coupled to the first portion (24) such that the reception space wall section (49) partly delimits the reception space (7) at a second side (50); and
a sample input cup (4) delimiting a sample input region (8) into which the sample is fillable,
wherein the sample input region (8) is in fluid communication with the reception space (7),
wherein the reception space wall section (49) and at least a portion of the sample input cup (4) are integrally formed,
wherein during normal operation for performing a measurement of the liquid sample, the arrangement has an orientation such that a longitudinal axis (26) of the sample input cup (4) is oriented substantially vertically and the sample input cup allows access to the sample input region (8) from above, via a sample input aperture (27) at a top portion of the sample input cup (4),
wherein an extent (D) of a cross section of the sample input region (8) perpendicular to the longitudinal axis (26) decreases from the top portion to the bottom portion of the sample input cup (4), the sample input region (8) being delimited by an inner face (28) of the sample input cup.

2. Arrangement according to the preceding claim,
wherein the sample input aperture (27) at the top portion of the sample input cup (4) allows insertion of a pipette or a capillary or a hollow needle, and/or
wherein the sample input cup (4) is substantially rotationally symmetric with respect to the longitudinal axis (26) of the sample input cup.

3. Arrangement according to one of the preceding claims,
wherein the sample input cup (4) is funnel shaped and/or bell shaped and/or has a continuous narrowing and/or continuous widening having an apex at a bottom portion, and/or
wherein the extent (D) of a cross section of the sample input region close to or at the bottom portion of the cup is smaller than a diameter of a water drop having volume between 10 µl and 50 µl, such that in particular water wets the inner face of the sample input cup at the bottom portion, and/or
wherein a diameter (D) of the sample input region (8) or sample input cup inner face (28) increases by a factor of greater than two from bottom to top of the sample input cup.

4. Arrangement according to one of the preceding claims,
wherein the sample input cup (4) comprises at least one side inlet (29, 30) and/or at least one side outlet (31), in particular including a connector tube, having its longitudinal direction (32) running in a direction including an angle (a) between 30° and 60°, in particular 40° and 50°, with the longitudinal direction (26) of the sample input cup (4),
in particular allowing to provide a sample manifold and/or inlet of working solution and/or inlet of calibration solution and/or inlet of washing solution and/or outlet of waste solution or overflow liquid.

5. Arrangement according to one of the preceding claims,
wherein the at least one side inlet (29, 30) is oriented and positioned relative to the sample input cup such that liquid supplied by the side inlet discharges substantially tangentially to the inner face (28) of the sample input cup (4) into the sample input cup; and/or
wherein the longitudinal direction (32) of the at least one side inlet (29) is parallel to a tangential at the inner face (28) of the sample input cup (4) and/or the side inlet (29, 30) is positioned such that liquid supplied by the side inlet into the sample input cup wets/streams at a portion of the inner face of the sample input cup immediately adjacent to a liquid communication opening between the side inlet and sample input cup; and/or
wherein a liquid communication opening (34) between the side inlet (30) and sample input cup (4) is above a liquid level (60) of a reference amount of liquid filled into the sample input cup (4).

6. Arrangement according to one of the preceding claims,
wherein the at least one side inlet comprises a first side inlet (29) and a second side inlet (30) arranged at different axial positions of the sample input cup, and in particular substantially at laterally opposite sides of the sample input cup (4),
wherein the first side inlet and a second side inlet in particular allow introduction of liquid into the sample input region in form of a spiral shaped stream at an inside surface of the sample input cup preventing cross-contamination and/or carry over and/or spill over and/or allowing cleaning or purging the inner face of the sample input cup, and/or
wherein the side outlet (31) includes an overflow outlet arranged axially above the first side inlet (29) and second side inlet (30).

7. Arrangement according to one of the preceding claims,
wherein the sample input cup (4) comprises an output aperture (35) at a bottom portion, in particular in a central region of the bottom portion,
wherein the second portion (22) comprises material (37) below the output aperture (35) configured to circumferentially delimit a, in particular straight, input conduit (36), in particular substantially oriented along a longitudinal axis (26) of the sample input cup, providing fluid communication between the sample input region (8) and the reception space (7),
wherein an outer surface (39) of material delimiting the input conduit serves as adherent surface at which the first portion (24) is attached, in particular glued.

8. Arrangement according to one of the preceding claims,
wherein the first portion (24) and the second portion (22) each comprise respective, in particular plane, interface surface regions (39, 51) provided to form adherent surfaces at which the first portion and the second portion are mounted, in particular glued, together, and/or
wherein the interface surface region (39) of the second portion is laterally offset from an input conduit axis (26) of the input conduit (36) towards the first side, in particular due to material (37) delimiting the input conduit at the first side.

9. Arrangement according to one of the preceding claims, wherein the sample input cup comprises at least one of the following zones:
a first zone (43), in particular at the bottom portion, comprising a continuously changing diameter, wherein an inner surface of the first zone includes in particular an angle between 10° and 60° with a longitudinal axis of the sample input cup;
a second zone (42), in particular at a middle portion, comprising a substantially constant diameter and having one or more inlets, wherein an inner surface of the second zone in particular includes an angle between 0° and 10° with a longitudinal axis of the sample input cup;
a third zone (41), in particular at a top portion, comprising a continuously changing diameter or one or more step wise changes of the diameter, wherein an inner surface of the third zone includes in particular an angle between 40° and 60° with a longitudinal axis of the sample input cup.

10. Arrangement according to one of the preceding claims,
wherein the second portion (22) is integrally formed, in particular manufactured by injection molding, further in particular using polycarbonate, polyester, polyamides and blends thereof, and/or
the second portion further comprising, in particular downstream the reception space:
a reception space output port (44) including a tube connector (45), and/or
wherein the measurement area (21) comprises at least one analyte sensitive area, in particular ion-selective electrode, and/or
wherein the arrangement including the sample input cup (4) is configured to support direct ion selective electrode measurements of undiluted sample as input via the sample input cup.

11. Arrangement according to the preceding claim, further comprising at least one of:
at least one suction pump (5), connected to the reception space output port;
a liquid column separator (9) connected to the suction pump, to receive liquid at an input port (10) and configured to output the liquid via an output port (11), in particular to a waste compartment (14), such that a continuous liquid column between the input port and the output port of the liquid column separator is interrupted.

12. Arrangement according to one of the preceding claims, the liquid column separator comprising:
at least one, in particular substantially vertically arranged, guiding member (52) having a guiding surface at which incoming liquid is collected and is guided to run downwards,
at least one drip off edge (53) at a bottom of the guiding member;
a bottom collection region (54) vertically spaced apart from and below the drip off edge,
wherein the liquid column separator is in particular formed by an upper member comprising the guiding member and formed by a lower member forming the bottom collection region.

13. Arrangement according to one of the preceding claims,
wherein for guiding the liquid two parallel and spaced apart, in particular transparent, guiding plates (56, 57) are provided between which the liquid is allowed to run downwards as a continuous liquid film, and/or
wherein an optical measurement is enabled in transmission through the transparent guiding plates (56, 57) and the liquid in between.

14. Method of measuring at least one constituent of a liquid sample (47), in particular comprising serum, plasma and/or urine, the method comprising:
filling the sample into a sample input region (8) delimited by a sample input cup (4);
partly delimiting a reception space (7) for the sample by a first portion (24) at a first side (40), wherein the first portion comprises at least one measurement area (21);
partly delimiting the reception space (7) at a second side (50) by a reception space wall section (49) of a second portion (22) coupled to the first portion (24);
wherein the sample input region (8) is in fluid communication with the reception space (7),
wherein the reception space wall section and the sample input cup are integrally formed;
the method in particular further comprising:
guiding the sample into the reception space (7); and
measuring the sample (47) in contact with the measurement area (21),
wherein a longitudinal axis (26) of the sample input cup (4) is oriented substantially vertically and the sample input cup allows access to the sample input region (8) from above, via a sample input aperture (27) at a top portion of the sample input cup (4),
wherein an extent (D) of a cross section of the sample input region (8) perpendicular to the longitudinal axis (26) decreases from the top portion to the bottom portion of the sample input cup (4), the sample input region (8) being delimited by an inner face (28) of the sample input cup.

15. Method of manufacturing an arrangement (1) for measuring at least one constituent of a liquid sample, the method comprising:
manufacturing a first portion (24) comprising at least one measurement area;
manufacturing a second portion (22) comprising a reception space wall section (49) and a sample input cup (4);
coupling the second portion (22) to the first portion (24)
such that the reception space (7) is delimited by the first portion (24) at a first side (40), and
such that the reception space (7) is partly delimited by the reception space wall section (49) at a second side (50);
wherein the sample input cup (4) is configured to delimit a sample input region (8) into which the sample is fillable,
wherein the sample input region (8) is in fluid communication with the reception space (7),
wherein the reception space wall section (47) and at least a portion of the sample input cup (4) are integrally formed,
wherein during normal operation for performing a measurement of the liquid sample, the arrangement has an orientation such that a longitudinal axis (26) of the sample input cup (4) is oriented substantially vertically and the sample input cup allows access to the sample input region (8) from above, via a sample input aperture (27) at a top portion of the sample input cup (4),
wherein an extent (D) of a cross section of the sample input region (8) perpendicular to the longitudinal axis (26) decreases from the top portion to the bottom portion of the sample input cup (4), the sample input region (8) being delimited by an inner face (28) of the sample input cup.
